# EUROPEAN PATENT SPECIFICATION

(11) **EP 1 728 474 B1**
(45) Date of publication and mention of the grant of the patent: **05.08.2009**
(21) Application number: 06011498.0
(22) Date of filing: 02.06.2006
(51) Int. Cl.: A61B 17/064, A61B 17/04

(54) **Rivet for tissue**
Gewebeniete
Rivet pour tissu

(30) Priority: 03.06.2005 US 687074 P
(43) Date of publication of application: 06.12.2006
(73) Proprietor: Tyco Healthcare Group LP, Norwalk, Connecticut 06856 (US)
(72) Inventor: Blier, Kenneth, Meriden, CT 06450 (US); Viola, Frank J, Sandy Hook, CT 06482 (US); Beetel, Robert J., Hamden, CT (US); Heinrich, Russell, Madison, CT 06443 (US); Soltz, Michael A., North Haven, CT 06473 (US)
(74) Representative: HOFFMANN EITLE

(56) References cited:
- WO-A-03/094750
- US-A1- 2001 005 475
- US-A1- 2002 026 216
- US-A1- 2003 069 603
- US-A1- 2004 030 351
- US-B1- 6 482 210

## Description

### BACKGROUND

### 1. Technical Field

The present disclosure relates generally to medical fasteners, more specifically, to a medical rivet for piercing tissue and inducing hemostatis thereon.

### 2. Background of Related Art

During surgical procedures and specifically during sealing of tissue a variety of medical fasteners, such as staples are used. Fasteners are generally applied using a surgical instrument, such as a stapler, which automates the sealing process. Therefore, use of fasteners is preferred in certain situations since application of fasteners takes less time than other tissue sealing methods (e.g., sutures).

Typically, fasteners must be of specific length to penetrate and seal tissue of corresponding thickness. Therefore these is a need for a fastener which will create hemostasis in tissue of varying thickness.

A stapling device is disclosed in US 2002/0026216 A1.

### SUMMARY

The invention is defined in claim 1 below. The dependent claims are directed to optional features. The present disclosure provides for a medical rivet for securing tissue having a first and second surfaces and creating hemostatis thereon. The rivet includes a shaft having a head at a proximal end and a tip at a distal end. The tip is configured to penetrate the tissue through both surfaces and to form a mushroomed tip or a modified B-formation tip on the second surface of the tissue to secure the tissue therebetween and the head which is in contact with the first surface. The diameter of the mushroomed tip and/or the modified B-formation tip varies depending on the thickness of the tissue. The rivet also includes one or more spring-like structures disposed between the head and a plate configured to be in contact with the first surface of the tissue. Upon formation of the mushroomed tip and/or the B-formation tip, the spring-like structures maintain a substantially constant pressure on the tissue regardless of the tissue's thickness.

According to one aspect of the present disclosure a system for sealing tissue having a first surface and a second surface is disclosed. The system includes one or more rivets having a shaft that has a proximal and a distal end. The rivet further includes a head at the proximal end and a tip at the distal end, wherein the tip has a shape suitable for tissue penetration and is adapted to penetrate tissue through the first and second surfaces. The system also includes an anvil having a depression adapted to interface with the tip and to modify the shape of the tip upon application of pressure thereto to secure tissue between the head which is in contact with the first surface and the tip which is contact with the second surface.

According to another aspect of the present disclosure, a medical rivet for sealing tissue having a first surface and a second surface is disclosed. The rivet includes a shaft having a proximal and a distal end, a head disposed at the proximal end of the shaft and a tip disposed at the distal end of the shaft. The tip has a shape suitable for tissue penetration and is adapted to penetrate tissue through the first and second surfaces. The shape of the tip is adapted to be modified upon application of pressure thereto to secure tissue between the head which is in contact with the first surface and the tip which is contact with the second surface.

According to a further aspect of the present disclosure a method for sealing tissue having a first surface and a second surface is disclosed. The method includes the steps of penetrating tissue through the first and second surface with at least one rivet. The rivet includes a shaft having a proximal and a distal end. The rivet further includes a head at the proximal end and a tip at the distal end. The method also includes the step of applying pressure to the tip to modify the shape of the tip and to secure tissue between the head which is in contact with the first surface and the tip which is contact with the second surface.

According to another aspect of the present disclosure, a medical rivet for sealing tissue having a first surface and a second surface is disclosed. The rivet includes a shaft having a proximal and a distal end, a head disposed at the proximal end of the shaft and a tip disposed at the distal end of the shaft. The tip has a shape suitable for tissue penetration and is adapted to penetrate tissue through the first and second surfaces. The rivet also includes two or more locking members adapted to secure a washer inserted onto the shaft thereby securing tissue between the head which is in contact with the first surface and the washer which is contact with the second surface.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a side view of a medical rivet according to the present disclosure;

Figs. 2A-B are cross-sectional views of the medical rivet of Fig. 1 penetrating tissue according to the present disclosure;

Fig. 3 is a top view of an anvil for use with the medical rivet of Fig. 1 according to the present disclosure;

Fig. 4 is a cross-sectional view of the anvil of Fig. 3 according to the present disclosure;

Fig. 5 is a cross-sectional view of an alternative embodiment of a medical rivet according to the present disclosure;

Fig. 6 is a side view of the medical rivet of Fig. 5 according to the present disclosure;

Figs. 7A-C are cross-sectional views of the medical rivet of Fig. 5 penetrating tissue according to the present disclosure; and

Fig. 8 is a cross-sectional view ofa medical rivet.

### DETAILED DESCRIPTION

Preferred embodiments of the present disclosure will be described herein below with reference to the accompanying drawings. In the following description, well-known functions or constructions are not described in detail to avoid obscuring the present disclosure in unnecessary detail.

The present disclosure provides for a rivet having a shaft with a head at a proximal end and a tip at a distal end. The rivet is configured to penetrate tissue wherein the head prevents the rivet from passing through the tissue and anchors the rivet at a first surface of the tissue. The tip is flattened and anchors the rivet at a second surface of the tissue (e.g., first surface being one layer of tissue and second surface being another layer of tissue) thereby creating hemostasis.

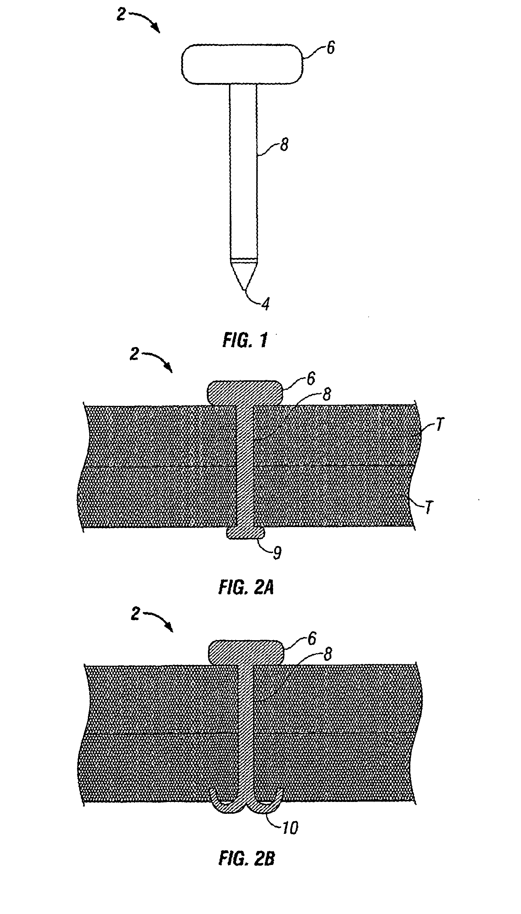

With reference to Fig. 1 a medical rivet 2 is shown having a shaft 8 with a head 6 at a proximal end and a tip 4 at a distal end. The shaft 8, the head 6, and the tip 4 of the rivet 2 are integrally formed from either biocompatible, bio-absorbable or medical grade metal (such as stainless steel, titanium, etc.), resin, polymeric or synthetic substance or combinations thereof of sufficient malleability so that the tip 4 is capable of being formed (e.g., split) under sufficient force.

The shaft 8 is preferably smooth and is cylindrical in shape. Those skilled in the art will appreciate that the shaft 8 can be granular (e.g., having a plurality of surfaces). The head 6 also has a cylindrical shape and has a larger diameter than the shaft 8 to prevent the rivet 2 from passing through the tissue intended for sealing. It is envisioned that the head 6 can have a variety of shapes, such as a dome, polygonal (e.g., hexagonal, octagonal, etc.). The tip 4 has a shape suitable for penetrating tissue, for example, the tip 4 may be tapered to allow for smoother penetration of tissue.

With reference to Figs. 2A-B the rivet 2 is shown penetrating tissue T. The shape of tip 4 is modified to ensure that tissue is secured between the head 6 and the tip 4. In Fig. 2A, the tip 4 has been formed into a mushroomed tip 9 by pressure applied thereto. To achieve the mushroomed tip 9 pressure is applied to the tip 4 at the center thereof to push tip 4 in all directions uniformly. The pressure can be applied by squeezing the rivet 2 between jaws of a forming instrument (not shown) wherein the pressure is applied on the tip 4 while the head 6 is supported to provide a counteractive force. Such a staple instrument is within the purview of those skilled in the art.

With reference to Fig. 2B, a cross-sectional view of the rivet 2 penetrating tissue T is shown where the tip 4 is split into a modified B-formation tip 10. To achieve B-formation tip 10, the pressure is applied along a center axis of the tip 4 to push tip 4 evenly in two opposite directions. The pressure can be applied using the same staple instrument discussed with regard to the mushroomed tip 9. It is also envisioned that the tip 4 may be split into three, four, five, etc. segments which may be split evenly or unevenly and/or pointing into any number of directions to achieve desired hemostatis.

Those skilled in the art will appreciate that both of the modified tips (e.g., the mushroomed tip 9 and the B-formation tip 10) can be used interchangeably to achieve a proper seal via the rivet 2. The mushroomed tip 9 or the B-formation tip 10 may be used with relatively and/or thicker tissue. Larger diameter of formed tips allows for reduction in the length of the shaft 8 thereby securing the rivet 2 to the tissue T.

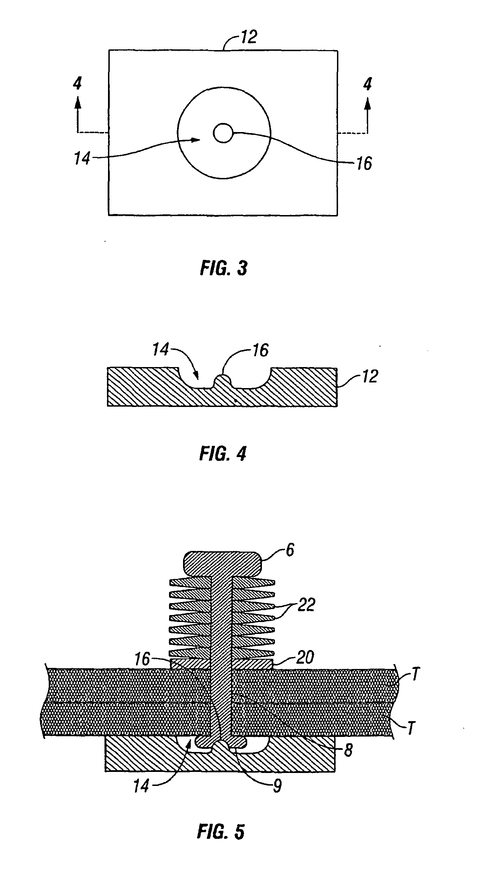

An anvil 12 for forming the mushroomed tip 9 and the B-formation tip 10 will now be discussed with reference to Figs. 3 and 4. Fig. 3 shows a top view of the anvil 12 and Fig. 4 shows a cross-sectional view thereof along a cross-section line 4. The anvil 12 includes a depression 14 having a depth and a border configured to fit around the tip 4. The depression 14 includes a splitter 16 positioned at the center of the depression 14. The splitter 16 is used to achieve certain configurations of the tip 4.

The depression 14 is shaped to interface with the tip 4 and is shaped to form the tip 4 into a desired modified form (e.g., mushroomed tip 9). In the illustrated embodiment, the depression 14 has a substantially round shape wherein the border limits expansion of the tip 4. The border and the depth of the depression 14 vary accordingly depending on the length of the shaft 8 and the thickness of tissue to be secured. Thus, as the tip 4 interfaces with the depression 14 and pressure is applied thereto the tip 4 is formed into a mushroomed tip 9, wherein the mushroomed tip 9 does not extend beyond the borders of the depression 14. The mushroom tip 9 may also be formed using the splitter 16, wherein the splitter 16 splits the tip 4 evenly in all directions. The shape of the splitter 16 depends on the desired shape modified tip. If the mushroomed tip 9 is to be achieved, then the splitter 16 has a point-like tip configured to split tip 4 in all directions. If the B-formation tip 10 is to be achieved, the splitter 16 has an edge-like tip configured to split the tip 4 substantially equally in only two directions. It is also envisioned that the splitter 16 can have various pyramidal shapes suitable for splitting the tip 4 into three, four, five, etc. segments which may be split evenly or unevenly and/or pointing into any number of directions to achieve desired hemostatis.

As shown in Fig. 5, the anvil 12 is brought into contact with the tip 4 of the rivet 2
wherein the splitter 16 forms the tip 4 into the mushroomed tip 9. A counteractive force is applied to the head 6 while pressure is applied by the anvil 12.

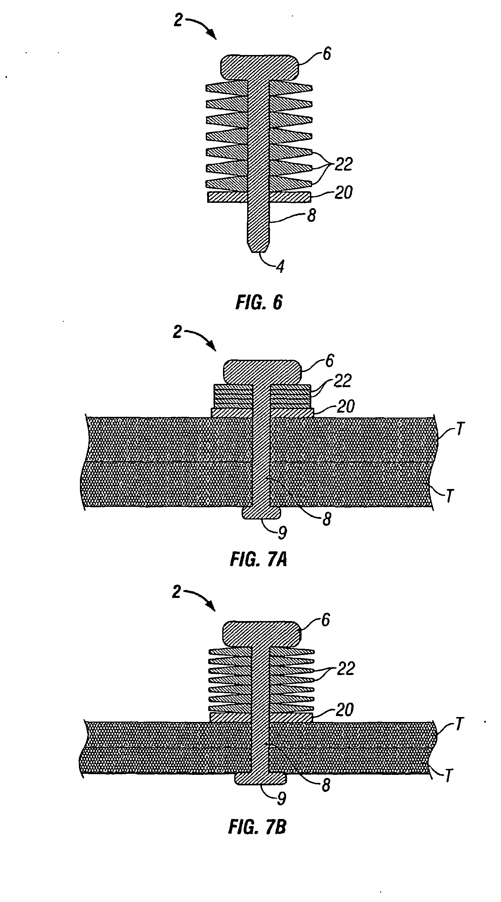

As seen in Figs. 5 and 6, an alternative embodiment of the rivet 2 shown, wherein the rivet 2 includes a plurality of Belleville washers 22 disposed between the head 6 and a spring plate 20. Belleville washers 22 are also known as a cupped spring washers, and are a type of non-flat washers. Washers 22 have a slight conical shape which gives them a spring characteristic. Washers 22 are used as springs, or to apply a pre-load or flexible quality to the rivet 2. Those skilled in the art will understand that other types of spring-like structures (e.g., springs) can be used to provide a flexible load quality to the rivet 2.

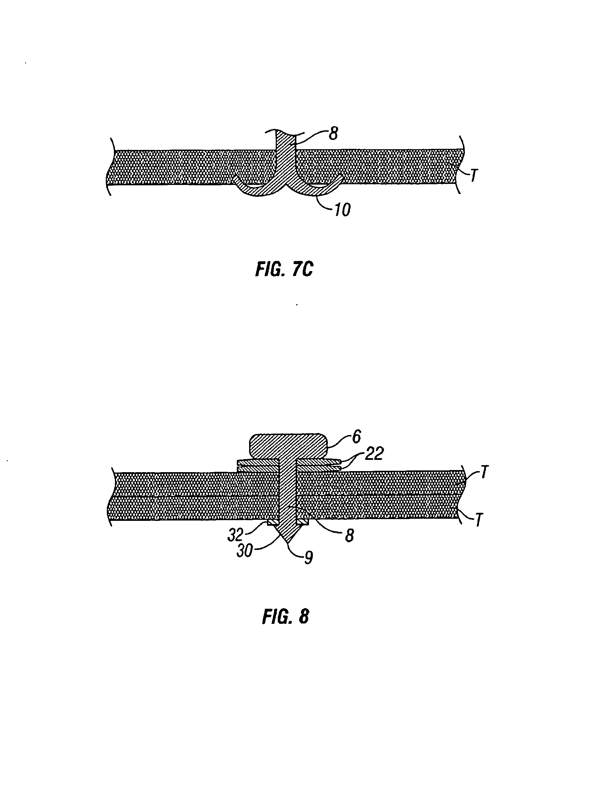

The washers 22 flatten, as shown in Fig. 7A, when compressed between the plate 20 and the head 6, thereby allowing the rivet 2 to penetrate thicker tissue T while maintaining sufficient pressure thereon. When the spacing between the head 6 and the plate 20 expands, the washers 22 attempt to revert to their original conical configuration continuing to maintain the pressure on the tissue T and securing the rivet 2 in place. The washers 22 maintain a substantially constant force on the tissue T over various thicknesses. In combination with the varying size and/or diameter of the formed tips (e.g., the mushroomed tip 9 and the B-formation tip 10) the rivet 2 can fit and secure tissue T of varying thickness. Other compression mechanisms may be used, such as springs, which maintain pressure on tissue after the rivet 2 is inserted therein. As seen in Fig. 7C, the mushroomed tip 9 can be substituted by the B-formation tip 10 as discussed above with reference to Figs. 2A-B.

Fig. 8 shows another medical rivet 6. The tip 9 is secured against the tissue T without deforming and/or modifying the tip 9. Instead, the medical rivet 6 is secured to the first and second surfaces of the tissue T after the rivet 6 penetrates the tissue by sliding a washer 32 onto the tip 9. The washer 32 is secured therein via two or more locking members 30. The locking members 30 may be protrusions or finger joints which are adapted to allow the washer 32 to slide in one way (e.g., toward the head 6) and not slide out once the washer 32 is inserted beyond the locking members 30. The locking members 30 may be placed anywhere along the length of the shaft 8 depending on the size of the tissue being secured. To facilitate various thickness of tissue one or more spring-like structures (e.g., Belleville washer 22) may be used to maintain a substantially constant force on the tissue T over various thicknesses.

## Claims

1. A system for sealing tissue having a first surface (T) and a second surface (T), the system comprising:
at least one rivet (2) including a shaft (8) having a proximal and a distal end, the rivet (2) further having a head (6) at the proximal end and a tip (4) at the distal end, wherein the tip (4) has a shape suitable for tissue penetration and is adapted to penetrate tissue through the first and second surfaces (T);
an anvil (12) **characterised in that** the anvil (12) includes a depression (14) having a splitter (16) that is adapted to interface with the tip (4) and to modify the shape of the tip (4) upon application of pressure thereto, wherein upon application of said pressure the tip (4) is split by the splitter (16) into a plurality of segments to secure tissue between the head (6) which is in contact with the first surface (T) and the tip (4) which is in contact with the second surface (T).

2. A system as in claim 1, wherein the shape of the tip (4) is such as to achieve a modified B-formation (10) upon application of said pressure.

3. A system as in any one of the preceding claims, wherein the at least one rivet (2) includes at least one spring-like structure (22) disposed between the head (6) and the tip (4).

4. A system as in claim 3, wherein the at least one spring-like structure (22) is a Belleville washer.

5. A system as in any one of the preceding claims, wherein the shaft (8), the head (6) and the tip (4) of said rivet are integrally formed.

6. A system as claimed in any one of the preceding claims, wherein said rivet is formed from a material selected from metal, resin, polymeric substance and synthetic substance.

## Patentansprüche

1. System zum Abdichten von Gewebe mit einer ersten Oberfläche (T) und einer zweiten Oberfläche (T), wobei das System umfasst:
zumindest einen Niet (2) mit einem Schaft (8), der ein proximales und ein distales Ende aufweist, wobei der Niet (2) ferner einen Kopf (6) an dem proximalen Ende und eine Spitze (4) an dem distalen Ende aufweist, wobei die Spitze (4) eine geeignete Form zum Durchdringen von Gewebe hat und angepasst ist, um Gewebe durch die erste und zweite Oberfläche (T) zu durchdringen;
einen Amboss (12), **dadurch gekennzeichnet, dass** der Amboss (12) eine Senke (14) mit einer Spalteinrichtung (16) enthält, die angepasst ist, die Spitze (4) zu berühren und die Form der Spitze (4) nach dem Anlegen von Druck daran zu modifizieren, wobei die Spitze (4) nach dem Anlegen des Drucks durch die Spalteinrichtung (16) in eine Mehrzahl von Segmenten aufgespalten wird, um Gewebe zwischen dem Kopf (6), der in Kontakt mit der ersten Oberfläche (T) ist, und der Spitze (4), die in Kontakt mit der zweiten Oberfläche (T) ist, zu befestigen.

2. System nach Anspruch 1, wobei die Form der Spitze (4) derart ist, um eine modifizierte B-Formation (10) nach dem Anlegen des Drucks zu erreichen.

3. System nach einem der vorstehenden Ansprüche, wobei der zumindest eine Niet (2) zumindest eine federähnliche Struktur (22) enthält, die zwischen dem Kopf (6) und der Spitze (4) angeordnet ist.

4. System nach Anspruch 3, wobei die zumindest eine federähnliche Struktur (22) eine Tellerfeder ist.

5. System nach einem der vorstehenden Ansprüche, wobei der Schaft (8), der Kopf (6) und die Spitze (4) des Niets integral ausgebildet sind.

6. System nach einem der vorstehenden Ansprüche, wobei der Niet aus einem Material gebildet ist, das aus Metall, Harz, Polymersubstanz und synthetischer Substanz ausgewählt ist.

## Revendications

1. Système pour l'obturation de tissu ayant une première surface (T) et une seconde surface (T) , le système comprenant:
au moins un rivet (2) incluant un arbre (8) ayant une extrémité proximale et une extrémité distale, le rivet (2) ayant en outre une tête (6) à l'extrémité proximale et une pointe (4) à l'extrémité distale, où la pointe (4) a une forme qui convient pour la pénétration dans le tissu et est apte à pénétrer dans le tissu à travers les première et seconde surfaces (T);
une enclume (12), **caractérisé en ce que** l'enclume (12) comprend un creux (14) ayant un organe de fendage (16) qui est apte à être en interface avec la pointe (4) et à modifier la forme de la pointe (4) lors de l'application d'une pression à celle-ci, où lors de l'application de ladite pression, la pointe (4) est fendue par l'organe de fendage (16) en une pluralité de segments pour fixer le tissu entre la tête (6) qui est en contact avec la première surface (T) et la pointe (4) qui est en contact avec la seconde surface (T).

2. Système selon la revendication 1, dans lequel la forme de la pointe (4) est réalisée de façon à atteindre une formation B modifiée (10) lors de l'application de ladite pression.

3. Système selon l'une quelconque des revendications précédentes, dans lequel le au moins un rivet (2) comprend au moins une structure (22) semblable à un ressort disposée entre la tête (6) et la pointe (4).

4. Système selon la revendication 3, dans lequel la au moins une structure (22) semblable à un ressort est une rondelle de Belleville.

5. Système selon l'une quelconque des revendications précédentes, dans lequel l'arbre (8), la tête (6) et la pointe (4) dudit rivet sont formés intégralement.

6. Système selon l'une quelconque des revendications précédentes, dans lequel ledit rivet est réalisé en un matériau sélectionné parmi le métal, la résine, la substance polymérique et la substance synthétique.
